# EUROPÄISCHE PATENTSCHRIFT

(11) **EP 1 207 859 B1**
(45) Veröffentlichungstag und Bekanntmachung des Hinweises auf die Patenterteilung: **22.09.2004**
(21) Anmeldenummer: 00947970.0
(22) Anmeldetag: 12.07.2000
(51) Int. Cl.: A61K 9/34, A61K 35/74, A61P 31/04

(54) **ORALE DARREICHUNGSFORM ENTHALTEND PROBIOTISCHE MIKROORGANISMEN**
ORAL FORM OF ADMINISTRATION CONTAINING PROBIOTIC MICRO-ORGANISMS
FORME DE PRESENTATION ORALE CONTENANT DES MICRO-ORGANISMES PROBIOTIQUES

(30) Priorität: 12.08.1999 DE 19937361
(43) Veröffentlichungstag der Anmeldung: 29.05.2002
(73) Patentinhaber: MERCK PATENT GmbH, 64293 Darmstadt (DE)
(72) Erfinder: BUG, Joachim, D-64291 Darmstadt (DE); MANNECK, Iris, D-65185 Wiesbaden (DE); SCHMID, Martina, D-71116 Gärtingen (DE)
(86) Internationale Anmeldenummer: PCT/EP2000/006580
(87) Internationale Veröffentlichungsnummer: WO 2001/012164

(56) Entgegenhaltungen:
- EP-A- 0 264 989
- WO-A-99/20745
- DATABASE WPI Week 8725 Derwent Publications Ltd., London, GB; AN 1987-288458 XP002155191 "Capsules containing bacteria dispersed in oil or powder" & JP 62 201823 A ((FREN) FREUND SANGYO KK) , 5. September 1987 (1987-09-05)
- DATABASE WPI Week 9727 Derwent Publications Ltd., London, GB; AN 1997-294858 XP002155192 "Preparation of granules containing useful enteric bacteria" & JP 09 110706 A ((HORI-N) HORIUCHI SHOKUHIN KOGYO KK), 28. April 1997 (1997-04-28)

## Beschreibung

Die Erfindung betrifft eine orale Darreichungsform, die wenigstens eine Gattung von probiotischen Mikroorganismen enthält, wobei sie selbst und/oder die probiotischen Mikroorganismen wenigstens einen magensaftresistenten Überzug aufweist bzw. aufweisen.

Viele Menschen, insbesondere in den wirtschaftlich und technisch hochentwickelten Ländern, klagen oft über temporäre oder chronische Verdauungsbeschwerden, die durch eine geschädigte oder gestörte Darmflora verursacht werden. Ursache für diese "Wohlstandserkrankungen" sind meist Streßsituationen, Medikamenten- oder Drogenmißbrauch, Folgeerscheinungen von Antibiotika-Behandlungen aber auch-sehr oft Fehlernährung.
Akute und drastische Beschwerden lassen sich mit bekannten Arzneimitteln, die nicht nur geeignete pharmazeutische Wirkstoffe, sondern auch entsprechende natürliche Enzyme oder darmspezifische Mikroorganismen beinhalten können, behandeln.

Bei ständigen, leichten, nicht direkt als Krankheit zu bezeichnenden Störungen des Intestinaltraktes ist hingegen oft schon der regelmäßige Verzehr von geeigneten, speziell ausgesuchten Lebensmitteln oder Nahrungsergänzungspräparaten auf Basis von probiotischen Mikroorganismen ausreichend, um die Symptome, die eine gestörte oder geschädigte Darmflora hervorrufen, zu lindem oder zu beseitigen. Aber auch bei intakter bzw. bei gesunder Darmflora kann die Zuführung von probiotischen Mikroorganismen, insbesondere in Verbindung mit Antioxidantien eine immunstimulierender Wirkung haben.

Joghurt- und Sauermilchprodukte erfreuen sich aus diesen Gründen einer zunehmenden Beliebtheit. Die meisten dieser für die Ernährung wertvollen Produkte, die geeignete probiotische Mikroorganismen zu diesem Zweck enthalten, sind jedoch Frischwaren und lassen sich daher nur unter Kühlung und dann auch nur wenige Tage aufbewahren.

Desweiteren gibt es auch Produkte, welche die geeigneten probiotischen Mikroorganismen als Monopräparat offerieren. Diese haben jedoch den Nachteil, daß sie in vielen Ländern nicht als Lebensmittel oder Lebensmittelergänzung zugelassen sind, da sie keine weiteren emährungsphysiologisch wertvollen Stoffe, wie z.B. Mineralstoffe, Fette, Vitamine, Kohlenhydrate, Eiweißstoffe, Ballaststoffe oder Spurenelemente enthalten.

Darüber hinaus können im Durchschnitt nur circa 10 % der eingenommenen probiotischen Mikroorganismen im menschlichen oder tierischen Darm ihre gesundheitsfördernde Aktivität entfalten. Daher ist es notwendig, eine wesentlich größere Menge probiotischer Mikroorganismen einzunehmen als es eigentlich therapeutisch notwendig ist, um eine ausreichend hohe Aktivität dieser probiotischen Mikroorganismen im menschlichen und tierischen Darm und damit auch eine gesundheitsfördemde Wirkung zu erzielen.

WO 99/20745 A1 offenbart ein mit einem magensaftresistenten Überzug ausgerüstetes Granulat enthaltend probiotische Milchsäurebakterien. Nach Herstellung des Granulats muss dieses jedoch einem weiteren Formulierungsschritt, wie Abfüllen in Kapseln oder in Sachets unterzogen werden, bevor es in einer zu Einnahme durch den Konsumenten geeigneten Form zur Verfügung steht. Beides ist jeweils mit einem weiteren Produktionsschritt und damit mit zusätzlichen Kosten verbunden. Bei Abfüllen in Sachets ergibt sich zudem bei deren Entnahme durch den Anwender noch das Problem von Produktverlusten, das einerseits auf üblicherweise in den Sachets verbleibender Restmengen und andererseits auf der vergleichsweise schwierigen Handhabung von Granulaten auf Grund von elektrostatischer Aufladung beruht.

JP 62201823A offenbart gegebenenfalls magensaftresistent überzogene Kapseln, die probiotische Mikroorganismen in ÖI dispergiert oder in einem Pulver verteilt enthalten.

Aufgabe der Erfindung war es daher eine Darreichungsform für probiotische Mikroorganismen bereitzustellen, die einfacher und somit kostengünstiger herzustellen ist und die sowohl eine einfachere Einnahme also eine präzisere Dosierung der Mikroorganismen ermöglicht.

Die Lösung der Aufgabe erfolgt erfindungsgemäß durch die Bereitstellung einer Tablette, die wenigstens eine Gattung von probiotischen Mikroorganismen enthält und bei der sie selbst und/oder die probiotischen Mikroorganismen wenigstens einen magensaftresistenten Überzug aufweisen.

Bevorzugt ist die Tablette eine Mehrschichttablette.

Als probiotische Mikroorganismen sind alle Mikroorganismen geeignet, die im gesunden menschlichen oder tierischen Darm entweder selbst üblicherweise vorkommen und/oder die eine gesundheitsfördernde Wirkung auf den gesunden, gestörten oder erkrankten Intestinaltrakt haben. Beispielsweise fördern probiotische Mikroorganismen die intestinale Lactoseverdauung bei Personen mit Milchzuckerunverträglichkeit oder die raschere Genesung bei verschiedenen Durchfallkrankheiten. Vorzugsweise handelt es sich bei den verwendeten probiotischen Mikroorganismen um Lactobacillen, Bifidobakterien oder Streptokokken. Besonders bevorzugt handelt es sich um Lactobacillus casei, Lactobacillus acidophilus, Bifidobakterium bifidum, Bifidobakterium longum und/oder Lactobacillus plantarum.

Die Menge der probiotischen Mikroorganismen in der erfindungsgemäßen Tablette ist so zu wählen, daß die angestrebte gesundheitsfördernde Wirkung gewährleistet wird. Vorzugsweise enthält die erfindungsgemäße Tablette 10³ bis 10¹², besonders bevorzugt 10⁵ bis 10¹¹ und ganz besonders bevorzugt 10⁷ bis 10¹⁰ probiotische Mikroorganismen. Für die Stabilität in Hinsicht auf die Anzahl und die Aktivität lebender Mikroorganismen ist es vorteilhaft wenn die verwendeten Materialien, insbesondere das Trägermaterial, in welches die probiotischen Mikrorganismen eingebettet sind, einen möglichst geringen Wassergehalt aufweisen. Vorzugsweise beträgt der Wassergehalt ≤ 3,0 Gew.%, besonders bevorzugt ≤ 0,1 Gew.% bezogen auf das Gewicht des Trägermaterials.

Erfindungsgemäß enthält die Tablette wenigstens einen magensaftresistenten Überzug. In einer bevorzugten Ausführungsform weist die erfindungsgemäße Tablette wenigstens einen Überzug auf, der im wesentlichen aus Schellack oder aus Schellack und Polyvinylpyrrolidon besteht.

In einer weiteren bevorzugten Ausführungsform weist die erfindungsgemäße Tablette wenigstens einen Überzug auf, welcher aus wenigstens zwei Schichten besteht, wobei eine Schicht im wesentlichen aus Hydroxypropylmethylcellulose, Methylcellulose und/oder Polyvinylpyrrolidon und/oder eine Schicht im wesentlichen aus Schellack oder Schellack und Polyvinylpyrrolidon besteht.

In einer weiteren bevorzugten Ausführungsform weist die erfindungsgemäße Tablette wenigstens einen Überzug auf, welcher aus wenigstens zwei übereinander angeordneten Schichten besteht, wobei die/eine innere, Kern-nahe Schicht im wesentlichen aus Hydroxypropylmethylcellulose, Methylcellulose und/oder Polyvinylpyrrolidon und/oder die/eine äußere, Kern-ferne Schicht im wesentlichen aus Schellack oder Schellack und Polyvinylpyrrolidon besteht.

Vorzugsweise enthält die erfindungsgemäße Tablette 1 bis 10 Gew.% Schellack, besonders bevorzugt 1,5 bis 6 Gew.% und ganz besonders bevorzugt 2 bis 3,5 Gew.%, bezogen auf das Gesamtgewicht der oralen Darreichungsform.

Für die erfindungsgemäße Tablette ist es wesentlich, daß sie einen magensaftresistenten Überzug aufweisen, der mindestens so groß ist, daß er die probiotischen Mikroorganismen vollständig umschließt.

Eine weitere bevorzugte Ausführungsform der Tablette enthält probiotische Mikroorganismen, die selbst mit einem magensaftresistenten Überzug versehen sind. Hierzu werden die probiotischen Mikroorganismen nach verschiedenen dem Fachmann bekannten Methoden getrocknet und anschließend mit mindestens einem magensaftresistenten Überzug versehen.

Darüber hinaus kann die erfindungsgemäße Tablette selbst und/oder die probiotischen Mikroorganismen zusätzlich zu dem/den magensaftresistenten Überzug/Überzügen gegebenfalls noch einen oder mehrere weitere(n) Überzug/Überzüge aufweisen. Vorzugsweise dient/dienen dieser Überzug/diese Überzüge dazu, eine bessere Anhaftung des/der magensaftresistenten Überzuges/Überzüge zu erzielen und/oder der Verbesserung des Geschmacks, der Haltbarkeit und/oder der optischen Erscheinung.

Die Überzüge können sowohl aus wäßriger Lösung, wie auch aus organischer Lösung aufgebracht werden. Für die erfindungsgemäße Tablette ist es von Vorteil, wenn der erste Überzug bzw. die erste oder innere, Kern-nahe Schicht aus organischer Lösung aufgebracht wird, da die probiotischen Mikroorganismen häufig sehr empfindlich auf Feuchtigkeit reagieren. Besonders vorteilhaft ist ein Aufbringen der Überzüge bzw. Schichten aus einer alkoholischen Lösung der Überzugsmaterialien.

In einer weiteren bevorzugten Ausführungsform enthält die erfindungsgemäße Tablette zusätzlich zu den probiotischen Mikroorganismen weitere ernährungsrelevante Zusätze. Vorzugsweise enthält sie Vitamine, Mineralstoffe, Spurenelemente, Ballaststoffe, Enzyme, Pflanzenextrakte, Eiweiße, Kohlenhydrate und/oder Fette. Enthält die orale Darreichungsform ernährungsrelevante Zusätze, deren Verdauung bereits im Magen beginnt, wie z.B. Eiweiße, so ist es wichtig, daß diese ernährungsrelevanten Zusätze zumindest nicht vollständig von einem magensaftresistenten Überzug umschlossen sind.

Hierbei kann es in Abhängigkeit von den eingesetzten ernährungsrelevanten Zusätzen notwendig sein, diese untereinander und/oder diese und die probiotischen Mikroorganismen so in die erfindungsgemäße Tablette einzubringen, daß sie nicht miteinander in Kontakt kommen. Vorzugsweise wird dies durch das Einbringen der ernährungsrelevanten Zusätze und/oder Mikroorganismen in verschiedene Schichten einer Mehrschichtablette erreicht.

Bevorzugte Vitamine sind Vitamin A (β-Carotin), Vitamin C, Vitamin E, Vitamine des B-Komplexes und/oder Vitamin K. Besonders bevorzugte Vitamine sind Vitamin A, Vitamin C und/oder Vitamin E. Die Menge an den Vitaminen richtet sich in der Regel nach der empfohlenen Mindestbedarfdosis für das jeweilige Vitamin, wobei diese jedoch auch um durchschnittlich 50 bis 200 % überschritten werden kann. Bevorzugte Bereiche sind für das Vitamin C zwischen 50 und 300 mg, für das Vitamin E 10 bis 50 mg, für das Vitamin A ≤ 1,5 mg und für die Vitamine des B-Komplexes 10 µg bis 20 mg.

Bevorzugte Mineralstoffe sind für den Verzehr geeignete, anorganische oder organische Natrium-, Kalium-, Calcium, Magnesium-, Zink- und/oder Eisensalze, weiche vorzugsweise als Carbonate, Bicarbonate, Phosphate, Biphosphate, Sulfate, Bisulfate, Chloride, Fluoride, Citrate und/oder Lactate vorliegen. Der Anteil an Mineralstoffen bezogen auf das Gesamtgewicht der oralen Darreichungsform beträgt vorzugsweise von 20 bis 40 Gew.%. Vorzugsweise enthält die erfindungsgemäße Tablette als Spurenelemente Silicium, Chrom, Mangan, Jod, Molybdän, Selen und/oder Kupfer.

Als Ballaststoffe enthält die erfindungsgemäße Tablette vorzugsweise Sojakleie, Maiskleie, Weizenkleie und/oder Getreideschrot, besonders bevorzugt Sojakleie. Der Anteil an Ballaststoffen bezogen auf das Gesamtgewicht der oralen Darreichungsform beträgt vorzugsweise 2 bis 50 Gew.%.

Bevorzugte Enzyme bzw. Co-Enzyme sind Lipasen und/oder Proteasen bzw. Co-Enzym Q, Superoxiddismutase und/oder Gluthathionperoxidase, die die Magenund/oder Darmfunktion und/oder den Stoffwechsel fördern. Diese können in an sich bekannter Menge und in an sich bekannter Form eingebracht werden.

Die Tablette enthält außerdem weitere probiotische Substanzen, vorzugsweise Oligo-Fructose und/oder andere Oligozucker.

Vorzugsweise sind die Pflanzenextrakte Trockenextrakte aus Echinaceae, Bioflavonoide, Polyphenole, Phytoöstrogene und/oder Saponine.

Vorzugsweise enthält die erfindungsgemäße Tablette als Eiweiße Sojaprotein und/oder Molkenprotein und/oder als Fette solche Fette, die mehrfach ungesättigte Fettsäuren enthalten.

Die erfindungsgemäße Tablette kann außerdem je nach Ausführungsform die üblichen Hilfs- und Zusatzsoffe enthalten. Die Auswahl der Hilfs- und/oder Zusatzstoffe hängt auch von den lebensmittelrechtlichen Bestimmungen des Landes ab, in dem die erfindungsgemäße Tablette zum Einsatz kommen soll. Vorzugsweise enthält die erfindungsgemäße Tablette als weitere Hilfsstoffe, insbesondere in dem Überzug, Weichmacher, wie z.B. Glycerin, Miglyol, Trennwax und/oder acetylierte Monoglyceride.
Als weitere Hilfs- und/oder Zusatzstoffe kommen für die erfindungsgemäßen Tabletten und/oder Mehrschichttabletten, Stärke (z.B. Maisstärke), Talkum, mikrokristalline Cellulose, Lactose, hochdisperses Siliciumdioxid, Polyvinylpyrrolidon und/oder Cellulosepulver zum Einsatz. Als weitere Bestandteile können als Bindemittel und/oder Trennmittel Kohlenhydrate, wie beispielsweise Mannit, Sorbit, Xylit, Glucose, Sucrose, Fructose, Maltose, Dextrose, Maltodextrin und/oder Kaolin und/oder Cellulosederivate, wie beispielsweise Methylcellulose, Hydroxypropylcellulose und/oder Hydroxypropylmethylcellulose und/oder Calciumcarbonat, Calcium-, Magnesiumund/oder Glycerinstearat eingesetzt werden. Desweiteren kann die erfindungsgemäße Tablette auch Farb-, Geschmacks- und/oder Aromastoffe, sowie Gleitmittel, Antioxidantien und/oder Stabilisatoren enthalten. Der Gehalt dieser Grundlagenstoffe richtet sich einerseits nach dem angestrebten Gehalt an probiotischen Mikroorganismen, Vitaminen, Enzymen, Ballaststoffen u.s.w. und andererseits nach Kriterien, die die mechanisch-physikalischen Eigenschaften der Tablette bestimmen, wie z.B. Härte, Verpreßbarkeit, Größe, Farbe und/oder Form.

Die Herstellung der erfindungsgemäßen Tablette kann nach verschiedenen dem Fachmann bekannten Methoden durchgeführt werden. Diese Methoden sind z.B. aus H. Sucker, P. Fuchs, P. Speisser, "Pharmazeutische Technologie", Stuttgart 1978 oder K.H. Bauer, K.H. Frömming, C. Führer, "Pharmazeutische Technologie", Stuttgart 1986 bekannt.

Ein weiterer Gegenstand der Erfindung sind Verfahren zur Herstellung einer erfindungsgemäßen Tablette, dadurch gekennzeichnet, daß der/die Überzug(e) aus wäßriger Lösung und/oder aus organischer Lösung, bevorzugt aus organischer Lösung und besonders bevorzugt aus alkoholischer Lösung aufgebracht wird/werden.

Die Überzüge können nach den üblichen dem Fachmann bekannten Verfahren, wie z.B. Dragieren, Aufsprühen von Lösungen, Dispersionen oder Suspensionen, durch Schmelzverfahren oder durch Pulverauftragsverfahren aufgebracht werden.

Die erfindungsgemäße Tablette hat den Vorteil, daß eine wesentlich geringere Menge an probiotischen Mikroorganismen notwendig ist, um die gewünschte gesundheitsfördernde Wirkung zu erzielen. Sie lassen sich daher wesentlich billiger herstellen.

### Beispiele:

Die folgenden Beispiele dienen der Erläuterung der Erfindung.

### Beispiel 1

Auf einer Exzenterpresse E1 der Firma Fette bzw. KS der Firma Kilian wurde eine Mischung aus 65 % Bakterienzubereitung, 6 % mikrokristalliner Cellulose, 20 % Tricalciumphosphat, 2 % Glycerylpalmitostearat, 0,6 % Magnesiumstearat, und 6,4 % Sprengmittel zusammen mit einer Vitamin- und einer Mineralstoffmischung zu einer oblongförmigen Tablette mit einem Kerngewicht von 1,35 g und den Maßen 20,0 mm x 8,8 mm x 7,0 mm verpreßt. Zur Herstellung des magensaftresistenten Überzuges wurde zunächst der Schellack unter Rühren in Ethanol gelöst und, nach Erhalt einer klaren Lösung, das Miglyol in die Lösung gegeben und für weitere 15 Minuten nachgerührt. Anschließend wurde diese Lösung mit Hilfe einer Schlick-Düse auf die Tablette gebracht. Die Prozeßparameter wurden so gewählt, daß ein homogener Filmauftrag erfolgte.
Die Menge an Schellack betrug 2,1 Gew.% bezogen auf das Gewicht des Kernes entsprechend 4,5 mg pro cm² Tablettenoberfläche.

### Beispiel 2

Auf einem Rundläufer der Firma Manesty wurde eine Mischung aus 10 % Bakterienzubereitung, 33 % Lactose, 48,4 % mikrokristalliner Cellulose, 2 % Glycerylpalmitostearat, 0,6 % Magnesiumstearat und 6,0 % Sprengmittel zusammen mit einer Vitamin- und einer Mineralstoffmischung zu einer eiförmigen Tablette mit einem Kerngewicht von 1,0 g und den Maßen 18,0 mm x 8,8 mm x 7,2 mm verpreßt. Im Anschluß daran wurde durch Aufsprühen aus ethanolischer Lösung ein Film aus Hydroxypropylmethylcellulose aufgebracht. Die Menge an aufgebrachter Hydroxypropylmethylcellulose betrug 0,8 Gew.% bezogen auf das Gewicht des Kernes, entsprechend 1,4 mg pro cm² Tablettenoberfläche. Auf diese erste Schicht aus Hydroxypropylrnethylcellulose wurde dann, ebenfalls durch Aufsprühen aus ethanolischer Lösung, ein weiterer, magensaftresistenter Überzug aus Schellack, Polyvinylpyrrolidon und acetylierten Monoglyceriden aufgebracht. Die Menge an aufgebrachtem Schellack betrug zwischen 0, 25 und 0,35 Gew.% bezogen auf das Gewicht des Kernes, entsprechend 4,5 mg/cm² bis 6,3 mg/cm² Tablettenoberfläche. Die Menge an acetylierten Monoglyceriden sowie an Polyvinylpyrrolidon betrug jeweils 14,2 Gew.% bezogen auf die eingesetzte Menge an Schellack.

### Beispiel 3

Auf einem Rundläufer der Firma Hata wurde eine Mischung aus 65 % Bakterienzubereitung, 6 % mikrokristalliner Cellulose, 20 % Tricalciumphosphat, 2 % Glycerylpalmitostearat, 0,6 % Magnesiumstearat, und 6,4% Sprengmittel zusammen mit einer Vitamin- und einer Mineralstoffmischung zu einer eiförmigen Tablette mit einem Kemgewicht von 1,35 g und den Maßen 21,0 mm x 10,0 mm x 8,0 mm verpreßt. Im Anschluß daran wurde durch Aufsprühen aus ethanolischer Lösung ein Film aus Hydroxypropylmethylcellulose und Glycerin oder Miglyol aufgebracht. Die Menge an aufgebrachter Hydroxypropylmethylcellulose betrug 0,8 Gew.% bezogen auf das Gewicht des Kernes, entsprechend 1,48 mg pro cm² Tablettenoberfläche. Die Menge an Glycerin bzw. Miglyol betrug 10 Gew.% bezogen auf die eingesetzte Menge an Hydroxypropylmethylcellulose. Auf diese erste Schicht aus Hydroxypropylmethylcellulose wurde, ebenfalls durch Aufsprühen aus ethanolischer Lösung, ein weiterer, magensaftresistenter Überzug aus Schellack, Polyvinylpyrrolidon und acetylierten Monoglyceriden aufgebracht. Die Menge an aufgebrachtem Schellack betrug zwischen 0,3 und 0,5 Gew.% bezogen auf das Gewicht des Kernes, entsprechend 4,1 mg/cm² bis 6,8 mg/cm² Tablettenoberfläche. Die Menge an acetylierten Monoglyceriden sowie an Polyvinylpyrrolidon betrug jeweils 14,2 Gew.% bezogen auf die eingesetzte Menge an Schellack.

## Patentansprüche

1. Tablette enthaltend wenigstens eine Gattung von probiotischen Mikroorganismen, **dadurch gekennzeichnet, daß** sie selbst und/oder die probiotischen Mikroorganismen wenigstens einen magensaftresistenten Überzug aufweist/aufweisen.

2. Tablette gemäß Anspruch 1, **dadurch gekennzeichnet, daß** sie eine Mehrschichttablette ist.

3. Tablette nach Anspruch 1 oder 2, **dadurch gekennzeichnet, daß** die probiotischen Mikroorganismen Lactobacillen, Bifidobakterien, oder Streptokokken, vorzugsweise Lactobacillus casei, Lactobacillus acidophilus, Bifidobakterium bifidum, Bifidobakterium longum und/oder Lactobacillus plantarum sind.

4. Tablette nach einem oder mehreren der Ansprüche 1 bis 3, **dadurch gekennzeichnet, daß** sie 10³ bis 10¹² probiotische Mikroorganismen enthält.

5. Tablette nach Anspruch 4, **dadurch gekennzeichnet, daß** sie 10⁵ bis 10¹¹ probiotische Mikroorganismen enthält.

6. Tablette nach Anspruch 5, **dadurch gekennzeichnet, daß** sie 10⁷ bis 10¹⁰ probiotische Mikroorganismen enthält.

7. Tablette nach einem oder mehreren der Ansprüche 1 bis 6, **dadurch gekennzeichnet, daß** der magensaftresistente Überzug im wesentlichen aus Schellack oder aus Schellack und Polyvinylpyrrolidon besteht.

8. Tablette nach einem oder mehreren der Ansprüche 1 bis 6, **dadurch gekennzeichnet, daß** der Überzug aus mindestens zwei Schichten besteht, wobei eine Schicht im wesentlichen aus Hydroxypropylmethylcellulose, Methylcellulose und/oder Polyvinylpyrrolidon und/oder eine Schicht im wesentlichen aus Schellack oder aus Schellack und Polyvinylpyrrolidon besteht.

9. Tablette nach Anspruch 8, **dadurch gekennzeichnet, daß** der Überzug aus mindestens zwei übereinander angeordneten Schichten besteht, wobei die/eine innere, Kern-nahe Schicht im wesentlichen aus Hydroxypropylmethylcellulose, Methylcellulose und/oder Polyvinylpyrrolidon und/oder die/eine äußere, Kern-ferne Schicht im wesentlichen aus Schellack oder aus Schellack und Polyvinylpyrrolidon besteht.

10. Tablette nach einem der Ansprüche 7 bis 9, **dadurch gekennzeichnet, daß** die Menge an Schellack 1 bis 10 Gew.% beträgt.

11. Tablette nach Anspruch 10, **dadurch gekennzeichnet, daß** die Menge an Schellack 1,5 bis 6 Gew.% beträgt.

12. Tablette nach Anspruch 11, **dadurch gekennzeichnet, daß** die Menge an Schellack 2 bis 3,5 Gew.% beträgt.

13. Tablette nach einem oder mehreren der Ansprüche 1 bis 12, **dadurch gekennzeichnet, daß** sie weitere emährungsrelevante Zusätze enthält.

14. Tablette nach Anspruch 13, **dadurch gekennzeichnet, dass** die ernährungsrelevante Zusätze Vitamine, Mineralstoffe, Spurenelemente, Ballaststoffe, Enzyme, Pflanzenextrakte, Eiweiße, Kohlenhydrate und/oder Fette sind.

15. Tablette nach einem oder mehreren der Ansprüche 1 bis 14, **dadurch gekennzeichnet, daß** sie weitere Hilfsstoffe enthält.

16. Tablette nach Anspruch 15, **dadurch gekennzeichnet, daß** sie die Hilfsstoffe in dem Überzug/den Überzügen enthält.

17. Tablette nach Anspruch 16, **dadurch gekennzeichnet, daß** die Hilfsstoffe Weichmacher sind.

18. Tablette nach Anspruch 17, **dadurch gekennzeichnet, daß** die Weichmacher Glycerin, Miglyol, Trennwax und/oder acetylierte Monoglyceride sind.

19. Verfahren zur Herstellung einer Tablette nach einem oder mehreren der Ansprüche 1 bis 18, **dadurch gekennzeichnet, daß** der Überzug aus wäßriger Lösung und/oder aus organischer Lösung, bevorzugt aus organischer Lösung, besonders bevorzugt aus alkoholischer Lösung aufgebracht-wird.

## Claims

1. Tablet comprising at least one species of probiotic microorganisms, **characterised in that** it itself and/or the probiotic microorganisms has/have at least one gastric juice-resistant coating.

2. Tablet according to Claim 1, **characterised in that** it is a multilayered tablet.

3. Tablet according to Claim 1 or 2, **characterised in that** the probiotic microorganisms are lactobacilli, bifidobacteria or streptococci, preferably Lactobacillus casei, Lactobacillus acidophilus, Bifidobacterium bifidum, Bifidobacterium longum and/or Lactobacillus plantarum.

4. Tablet according to one or more of Claims 1 to 3, **characterised in that** it contains from 10³ to 10¹² probiotic microorganisms.

5. Tablet according to Claim 4, **characterised in that** it contains from 10⁵ to 10¹¹ probiotic microorganisms.

6. Tablet according to Claim 5, **characterised in that** it contains from 10⁷ to 10¹⁰ probiotic microorganisms.

7. Tablet according to one or more of Claims 1 to 6, **characterised in that** the gastric juice-resistant coating essentially consists of shellac or of shellac and polyvinylpyrrolidone.

8. Tablet according to one or more of Claims 1 to 6, **characterised in that** the coating consists of at least two layers, where one layer essentially consists of hydroxypropylmethylcellulose, methylcellulose and/or polyvinylpyrrolidone and/or one layer essentially consists of shellac or of shellac and polyvinylpyrrolidone.

9. Tablet according to Claim 8, **characterised in that** the coating consists of at least two layers arranged one on top of the other, where the/an inner layer close to the core essentially consists of hydroxypropylmethylcellulose, methylcellulose and/or polyvinylpyrrolidone and/or the/an outer layer remote from the core essentially consists of shellac or of shellac and polyvinylpyrrolidone.

10. Tablet according to one of Claims 7 to 9, **characterised in that** the amount of shellac is from 1 to 10% by weight.

11. Tablet according to Claim 10, **characterised in that** the amount of shellac is from 1.5 to 6% by weight.

12. Tablet according to Claim 11, **characterised in that** the amount of shellac is from 2 to 3.5% by weight.

13. Tablet according to one or more of Claims 1 to 12, **characterised in that** it comprises further nutrition-relevant additives.

14. Tablet according to Claim 13, **characterised in that** the nutrition-relevant additives are vitamins, mineral substances, trace elements, dietary fibre, enzymes, vegetable extracts, proteins, carbohydrates and/or fats.

15. Tablet according to one or more of Claims 1 to 14, **characterised in that** it comprises further adjuvants.

16. Tablet according to Claim 15, **characterised in that** it comprises the adjuvants in the coating(s).

17. Tablet according to Claim 16, **characterised in that** the adjuvants are plasticisers.

18. Tablet according to Claim 17, **characterised in that** the plasticisers are glycerol, Miglyol, separating wax and/or acetylated monoglycerides.

19. Process for the production of a tablet according to one or more of Claims 1 to 18, **characterised in that** the coating is applied from aqueous solution and/or from organic solution, preferably from organic solution, particularly preferably from alcoholic solution.

## Revendications

1. Comprimé comprenant au moins une espèce de microorganismes probiotiques, **caractérisé en ce que**, lui-même et/ou les microorganismes probiotiques a/ont au moins un enrobage résistant aux sucs gastriques.

2. Comprimé selon la revendication 1, **caractérisé en ce qu'**il est un comprimé multi-couche.

3. Comprimé selon la revendication 1 ou 2, **caractérisé en ce que** les microorganismes probiotiques sont des lactobaciles, des bifido-bactéries ou des streptocoques, de préférence Lactobacillus casei, Lactobacillus acidophilus, Bifidobacterium bifidum, Bifidobacterium longum et/ou Lactobacillus plantarum.

4. Comprimé selon une ou plusieurs des revendications 1 à 3, **caractérisé en ce qu'**il contient 10³ à 10¹² microorganismes probiotiques.

5. Comprimé selon la revendication 4, **caractérisé en ce qu'**il contient 10⁵ à 10¹¹ microorganismes probiotiques.

6. Comprimé selon la revendication 5, **caractérisé en ce qu'**il contient 10⁷ à 10¹⁰ microorganismes probiotiques.

7. Comprimé selon une ou plusieurs des revendications 1 à 6, **caractérisé en ce que** l'enrobage résistant aux sucs gastriques est constitué essentiellement par une laque ou par une laque et polyvinylpyrrolidone.

8. Comprimé selon une ou plusieurs des revendications 1 à 6, **caractérisé en ce que** l'enrobage est constitué par au moins deux couches, dans lequel une couche est essentiellement constituée par hydroxypropylméthylcellulose, méthylcellulose et/ou polyvinylpyrrolidone et/ou une couche est essentiellement constituée par une laque ou par une laque et polyvinylpyrrolidone.

9. Comprimé selon la revendication 8, **caractérisé en ce que** l'enrobage est constitué par au moins deux couches agencées l'une sur l'autre, dans lequel la/une couche interne proche du coeur est essentiellement constituée par hydroxypropylméthylcellulose, méthylcellulose et/ou polyvinylpyrrolidone et/ou la/une couche externe éloignée du coeur est essentiellement constituée par une laque ou par une laque et polyvinylpyrrolidone.

10. Comprimé selon une ou plusieurs des revendications 7 à 9, **caractérisé en ce que** la quantité de laque est de 1 à 10% en poids.

11. Comprimé selon la revendication 10, **caractérisé en ce que** la quantité de laque est de 1,5 à 6% en poids.

12. Comprimé selon la revendication 11, **caractérisé en ce que** la quantité de laque est de 2 à 3,5% en poids.

13. Comprimé selon une ou plusieurs des revendications 1 à 12, **caractérisé en ce qu'**il comprend en outre des additifs concernant la nutrition.

14. Comprimé selon la revendication 13, **caractérisé en ce que** les additifs concernant la nutrition sont des vitamines, des substances minérales, des oligoéléments, des fibres diététiques, des enzymes, des extraits végétaux, des protéines, des hydrates de carbone et/ou des matières grasses.

15. Comprimé selon une ou plusieurs des revendications 1 à 14, **caractérisé en ce qu'**il comprend des adjuvants supplémentaires.

16. Comprimé selon la revendication 15, **caractérisé en ce qu'**il comprend les adjuvants dans le ou les enrobage(s).

17. Comprimé selon la revendication 16, **caractérisé en ce que** les adjuvants sont des plastifiants.

18. Comprimé selon la revendication 17, **caractérisé en ce que** les plastifiants sont glycérol, Miglyol, une cire de libération et/ou des mono-glycérides acétylatés.

19. Procédé pour la production d'un comprimé selon une ou plusieurs des revendications 1 à 18, **caractérisé en ce que** l'enrobage est appliqué à partir d'une solution aqueuse et/ou d'une solution organique, de préférence à partir d'une solution organique, en particulier de préférence à partir d'une solution alcoolique.
